# EUROPEAN PATENT APPLICATION

(11) **EP 1 857 073 A1**
(43) Date of publication of application: **21.11.2007**
(21) Application number: 07009914.8
(22) Date of filing: 18.05.2007
(51) Int. Cl.: A61F 2/06, A61L 27/44, A61L 29/18, A61L 31/18

(54) **Radiopaque compositions, articles and methods of making and using the same**

(30) Priority: 19.05.2006 US 437763
(71) Applicant: UPCHURCH SCIENTIFIC, INC., Oak Harbor, WA 98277 (US)
(72) Inventor: Bailey, Michael L, Oak Harbor Washington 98277 (US); Swett, James E, Oak Harbor Washington 98277 (US)
(74) Representative: Rees, Kerry

(57) **Abstract**

A radiopaque composition and implant comprising a mixture of a polymer, such as polyetherketone (PEEK), polyetherketone (PEK), polyetherketoneketone (PEKK), polyetherimide, or polyphenylsulfone, and bismuth trioxide (BiO₃) is provided. The mixture comprises BiO₃ of about twenty to thirty percent (20% to 30%) by weight. The composition is useful in radiopaque implants for mammals including humans, such as in catheters, stents, screws, anchors, dental caps and posts, bone and joint replacements, and the like. The composition is also useful for radiopaque markers in implants and for radiopaque articles used in surgical and other in vivo procedures. The radiopaque items can be easily identified and located in a patient's body via X-ray examination or fluoroscopy.

## Description

### Field of the Invention

This invention relates generally to certain radiopaque compositions and articles, and methods of using the same. More particularly, the invention relates to radiopaque compositions comprising BiO₃ and articles made therefrom and methods of using the same in connection with implants, stents, and articles used in the healthcare and medical fields.

### Background of the Invention

Medical technology has advanced greatly over the years. Among other things, the use of implants in many areas of the human body is now conventional. Such implants can be spinal cages and tillers used for a long term basis within a human body. Such implants can also be used in connection with dental surgery and dental applications, such as in fillings in teeth, root canals, and the like. In addition, the use of various types of fasteners, such as screws, to hold bones together or to hold foreign objects in place in or on a human body is conventional. Similarly, the use of a wide variety of implants, such as catheters or stents, like those used in vascular and other applications, is conventional. For example, U.S. Patent No. 6,517,571, issued to Brauker et al. on February 11, 2003, which is hereby incorporated by reference as if fully set forth herein, discloses a vascular graft made of polytetrafluoroethylene paste extrudate that has been expanded.

In addition to the use of implants, medical technology has advanced such that the use of various imaging systems during surgery and other procedures is conventional. For example, the use of X-ray devices, MRI devices, fluoroscopes, and the like is conventional for assisting an operator, such as a physician, nurse or technician, to precisely locate and to identify a tube, catheter, or other device within a patient's body during the surgery or other procedure. For example, U.S. Patent No. 6,463,317, issued October 8, 2002, to Kucharczyk et al., which is hereby incorporated by reference as if fully set forth herein, discloses a device and method for treating an aneurysm during which the device is inserted into a patient and the device's location in the patient's body is viewed via X-ray, fluoroscopy, and/or magnetic resonance imaging techniques.

There are many situations in which the use of radiographically opaque (radiopaque) materials is desired. Radiopaque materials can be easily identified and precisely located by X-ray examination. In some situations, it is desirable to have a radiopaque item which is nonetheless optically transparent so that fluid flow though the device can be viewed.

In some conventional applications, an entire implant may be radiopaque. In other situations, however, it may be desirable to use discrete radiopaque markers of a particular size or shape, or it may be desirable to use a radiopaque marker in a particular location of the implant. For example, U.S. Patent No. 6,340,367, issued January 22, 2002 to Stinson, et al., which is hereby incorporated by reference as if fully set forth herein, discloses a temporary and retrievable radiopaque marker for use on an implantable endoprosthesis, such as a stent, graft, or stent-graft (as may be used in coronary angioplasty). Similarly, in Stinson et al., U.S. Patent No. 6,626,936, issued on September 30, 2003, which is hereby incorporated by reference as if fully set forth herein, a bioabsorbable radiopaque marker for use on an implanted endoprosthesis such as a stent is disclosed. Still another example of a radiopaque marker used in an implant is provided by Carter, U.S. Patent No. 4,863,470, issued September 5, 1989, which is incorporated by reference as if fully set forth herein, in which a radiopaque tab indicating size made from a combination of silicone and either barium sulfate or bismuth trioxide is included in a breast implant.

In connection with medical and healthcare applications, the use of barium sulfate (BaSO₄) is conventional. Barium sulfate can be used as a powder to coat various materials that are then placed in the human body; when an X-ray is taken, the materials so coated show up clearly in the X-ray. Among other things, barium sulfate can be added to another material during fabrication such that the resulting product is radiopaque. For example, barium sulfate may be added to a polymeric material from which an implant, such as a spinal cage, is fabricated. The resulting spinal cage is radiopaque, thus allowing the easy identification and precise location of the spinal cage when implanted in a body. However, the density and properties of barium sulfate are such that significant amounts of barium sulfate are often needed to obtain the desired amount of radiopacity. Adding significant amounts of barium sulfate to a polymeric material can result in a weaker structure that does not have the desired strength.

The use of various polymers for implants, especially tubular-shaped implants, is conventional. For example, U.S. Patent No. 6,623,823, issued on September 23, 2003, to Onwumere, which is hereby incorporated by reference as if fully set forth herein, discloses radiopaque catheters and stents, including a polyurethane tubing coated with a radiopaque brominated polyurethane coating composition.

The use of polyetheretherketone (PEEK) for implants and medical devices is conventional. PEEK exhibits excellent properties of strength as well as biocompatibility. PEEK materials, including implants and PEEK devices for medical applications are available from a number of commercial sources, such as Invibio, Inc. of Greenville, South Carolina. However. PEEK, by itself, is not radiopaque. Hence, implants and medical devices made from PEEK do not show up very well in X-ray images. Thus, the precise location and identification of a PEEK implant from an X-ray can be very difficult.

Using devices made from PEEK with barium sulfate (BaSO₄) has been tried. Although such devices can he radiopaque due to the addition of barium sulfate to PEEK, the addition of the necessary amounts of barium sulfate for the desired radiopacity to the composition tends to weaken the strength of the resulting device. Thus, the use of an implant made from a composition of barium sulfate and PEEK provides an undesirable tradeoff between the desired mechanical properties of the device and its radiopacity.

### Summary of the Invention

The present invention solves a number of problems by providing, among other things, a radiopaque implant made from a radiopaque composition comprising a polymer such as polyetheretherketone (PEEK) and bismuth trioxide (BiO₃). In one embodiment, the implant can be made from a composition comprising PEEK and bismuth trioxide that can contain up to about thirty percent (30%) by weight bismuth trioxide. In another embodiment, a radiopaque implant is provided, which is made from a composition comprising PEEK and bismuth trioxide that contains an amount of bismuth trioxide that is in the range of about twenty percent (20%) to thirty percent (30%) by weight. In still other embodiments, medical devices such as catheters, stents, screws, dental fillings, and the like can be made from a composition containing PEEK and at least about ten percent (10%) bismuth trioxide. In yet another embodiment of the invention, an implantable item is provided which has one or more radiopaque markers. In yet another embodiment of the invention, an implant for veterinary purposes for implantation into animals (such as but not limited to mammals other than humans) is provided. In still other embodiments of the invention, certain other polymers, such as polyetherketone, polyetherketoneketone, polyphenylsulfone, and polyetherimide, can be used instead of PEEK.

It is an object of the invention to provide a radiopaque implant which is easy to identify and locate via X-ray examination or fluoroscopy.

It is another object of the invention to provide a radiopaque implant which provides the desired amount of radiopacity while still providing sufficient strength.

It is another object of the invention to provide a radiopaque medical device which allows a user to easily identify and locate the device within a patient's body via X-ray examination or fluoroscopy.

These and other objects of the invention will be apparent to those of skill in the art from the figures and detailed description which follow.

### Summary of the Drawings

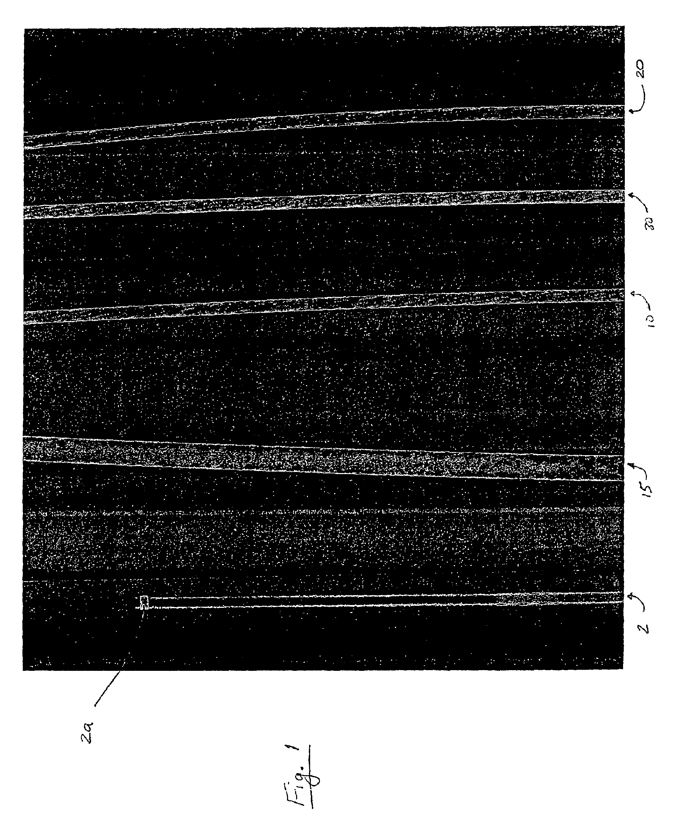

Figure 1 is a radiograph showing an X-ray image of various tubes, including tubes made in accordance with the present invention.

### Detailed Description of the Invention

Referring now to Fig. 1, a radiograph taken with conventional X-ray equipment is shown. These X-ray images were taken at 70KV for 15 seconds at 15mA. In Fig. 1, several different tubes 2, 10, 20, and 30 are shown. Tube 2 is a tube made of polyetheretherketone (PEEK). Tube 2 has a band 2a in Fig. 1, with the band 2a the X-ray image of a metal band placed around the exterior of tube 2. As those skilled in the art will appreciate, the metal band can be made of any metal that is radiopaque.

Still referring to Fig. 1, tube 10 is made from a mixture of PEEK and bismuth trioxide (BiO₃), in which the BiO₃ constitutes about ten percent (10%) by weight of the total composition from which tube 10 is fabricated. Similarly, tube 15 is also made from a composition consisting of PEEK and BiO₃ with the BiO₃ being ten percent (10%) by weight of the composition. As shown in Fig. 1, tube 15 is slightly larger in diameter than tube 10. In Fig. 1, tube 20 is made from a mixture of PEEK and BiO₃ in which the BiO₃ constitutes about twenty percent (20%) by weight of the total composition from which the tube 20 is fabricated. In Fig. 3, tube 30 is made from a mixture of PEEK and BiO₃ in which the BiO₃ constitutes about thirty percent (30%) by weight of the total composition from which tube 30 is fabricated. As is shown in Fig. 1, the tube 20 and tube 30 provide easily identifiable images in the radiograph. A comparison of the images of tube 20 and tube 30 with the tube 2 having a metal band shows that the tubes 20, 30 are sufficiently radiopaque for easy identification and location when tubes 20, 30 are implanted (either permanently or temporarily, such as during a surgical or other medical procedure, or when used as a catheter) within a body. Moreover, the images of tube 20 and tube 30 show sufficient transparency that the flow of fluids therethrough can be observed.

Besides tubing (such as shown in Fig. 1), other articles can be made in accordance with the invention. For example, the composition of PEEK and BiO_{3;} with the amount of BiO₃ by weight being in the range of from about twenty percent (20%) to about thirty percent (30%), can be used to fabricate rodstock in various sizes, as well as square profile rods, and monofilaments in various shapes and sizes. Such rodstock, square profile, and monofilament articles can be machined further to obtain desired shapes and sizes, such as for use as components by subsequent users of the base shape for such users' own designs or applications. For example, such monofilaments can be used to form sutures for use to close wounds or surgical incisions in a patient's body.

Those skilled in the art will appreciate that the PEEK and BiO₃ may be obtained from a number of commercial sources. For example, PEEK may be commercially obtained from Invibio, Inc. of Greenville, South Carolina, and BiO₃ may be commercially obtained from various sources. We have obtained such materials from a compounder, Fosters Corp., of Putnam Connecticut. The PEEK may be obtained in different colors and pellet sizes, and the BiO₃ may be obtained in a powder form. The BiO₃ powder may be mixed with the PEEK pellets, and the mixture may then be heated and further mixed to provide the desired composition containing the mixture of the BiO₃ in the PEEK. The resulting composition can then be molded to provide implants of a desired size and shape. Such implants can include spinal cages, screws, stents, and the like. In addition, those of skill in the art will appreciate that the resulting composition can alternatively be extruded, such as in a conventional manner for PEEK alone. Extrusion may be particularly useful in forming tubes made of the desired composition, including catheters, stents, and other implants. In addition, such articles can be machined to obtain desired shapes and sizes.

Besides tubing, catheters, grafts, and stents, the composition comprising PEEK and BiO₃ can be used for a wide variety of implants for use in the human body. Among other things, the PEEK and BiO₃ composition as described above can be used for any of the following types of implants: spinal cages, suture anchors, spiked washers, surgical screws, femoral implants, balloons, intracardiac pumps, heart valves, finger joints, hip and femoral bone replacements, bone screws and pins, dental posts and caps, and so forth.

PEEK provides a number of advantages. PEEK is biocompatible and biostable. PEEK articles may be easily sterilized, a conventional requirement for articles to be used in vivo with human patients. PEEK has inherent lubricity. PEEK is extremely strong - carbon fibers can be added to PEEK in order to obtain an article with mechanical properties of strength substantially the same as those of healthy human bones. PEEK typically has a melting point of about 343°C, a crystallization peak of about 160°C, and a glass transition temperature of about 145°C. PEEK can be extruded and injection molded by conventional techniques.

Those skilled in the art will also understand and appreciate that, depending on the particular application involved, other alternative polymers may be used in place of PEEK. Those skilled in the art will appreciate that in place of PEEK, other polyaryletherketones may be used as the polymer component of the composition. For example, the polymer commercially available under the trademark PARMAX, which is commercially available from Solvay Advanced Polymers of Alpharetta, Georgia, has appropriate mechanical and chemical properties such that it can be mixed with BiO₃ to obtain a radiopaque implant with the desired characteristics. PARMAX is a self-reinforcing polymer that is an amorphous, melt-processabie engineering thermoplastic. Other appropriate polymers include polyetherketone, polyetherketoneketone, polyphenylsufone, polyetherimide, and the polymers commercially available under trademarks RADEL R, which is also commercially available from Solvay Advanced Polymers, and ULTEM (polyetherimide), which is commercially available from the General Electric Company of Schenectady, New York.

Those skilled in the art will also appreciate that other polymers may be used in accordance with the invention. Such other polymers may include liquid crystal polymers, polyphthalamides, and polyarylamides. These latter polymers are either self-reinforcing polymers or can be reinforced with glass or carbon fibers. Each of these latter polymers are biocompatible in connection with short term blood contact.

In addition to the above polymers, the invention in another embodiment involves the use of a composition of polytetrafluoroethylene (PTFE) and BiO₃. The composition can be prepared such as described above, such as with BiO₃ in the amount by weight of from about twenty percent (20%) to about thirty percent (30%), by mixing PTFE and BiO₃ in powder form. The mixture can then be fabricated using conventional paste extrusion techniques and equipment. For example, the mixture of PTFE and BiO₃ can be combined with a liquid to disperse the PTFE and BiO₃ powder mixture, the dispersion blend may be extruded, and then the liquid may be removed, such as by drying or sintering. Those skilled in the art will appreciate that the PTFE and BiO₃ need not be mixed together in powder form first, but can be added to the liquid one after another.

Those skilled in the art will appreciate that implants made in accordance with the present invention may be either short term or long term implants. Indeed, articles made in accordance with the invention need not be implanted, but may be inserted into a patient's body for use during surgery or other medical procedures, then removed from the patient's body when the surgery or other procedure is finished. (Although regulations of the U.S. Food & Drug Administration (F.D.A.) define an "implant" as a "device that is placed into a surgically or naturally formed cavity of the human body if it is intended to remain there for a period of 30 days or more," those skilled in the art will appreciate that "implant" as used herein is also intended to include even those articles temporarily placed into a human body, as well as in any other animal body, including those of mammals other than humans.) "Implant" as used herein is intended to include any device which is inserted wholly or partially into a subject's body cavity (whether the subject is human or not), including those types of devices deemed an implant pursuant to F.D.A. regulations, such as those found in 21 C.F.R., Parts 870, 872, 874, 876, 878, 880, 882, 886, and 888 (as of April 2005). Those skilled in the art will also understand and appreciate that, although most of the foregoing discussion involves human patients, the articles and implants made in accordance with the invention may be useful in other areas as well, including in veterinary situations in which the patient is a cow, dog, cat, horse, or other mammal and not a human patient.

Radiopaque articles made in accordance with the present invention may be used in a variety of situations. As noted, such articles may be used as implants in a variety of applications. Such implants may be either short term implants or long term implants. In addition to use as implants, however, articles made in accordance with the invention may be used during surgical and other medical procedures. For example, a catheter (not shown) may be provided that has been made from a composition of PEEK and BiO₃, with about thirty percent (30%) by weight of the composition consisting of BiO₃. The catheter may be formed as a tube of a desired inner diameter size and outer diameter size. In addition, the catheter so formed may be of a desired length. Such a catheter in accordance with the invention may be used by inserting it into a patient's body, such as into an incision made to allow drainage from within the body. In addition, such a catheter may be inserted into a blood vessel and then guided by an operator (such as a physician) to a desired location, such as a blocked blood vessel in connection with coronary angioplasty. During the procedure, the operator can view a fluoroscope of a conventional type in order to identify and precisely locate the catheter as it is guided to the desired location in the patient's body. Once the operation is completed, the operator can then remove the catheter from the patient's body. Besides applications in coronary surgery, such a catheter may be used in any type of surgery in which the precise location of a catheter is desired, such as in neurological, ophthalmic, cosmetic, and dental surgeries, among others. In such situations, the use of a radiopaque article made in accordance with the present invention will be advantageous, as doing so allows the operator to determine the precise location of the article within the patient's body via the use of conventional X-ray, fluoroscopy, and/or magnetic resonance equipment. In addition, articles made in accordance with the present invention also are advantageous in that they may be fabricated to varying degrees of radiopacity. Referring back to Fig. 1, it can be scen that tube 30 (which was made from a composition consisting of thirty percent (30%) by weight BiO₃ and the remainder PEEK) is not totally radiopaque, but is sufficiently so to allow easy identification and location via an X-ray examination. At the same time, however, the tube 30 is sufficiently radiolucent that an operator may be able to view the movement of fluids or other items through tube 30 via X-ray examination or fluoroscopy with conventional equipment. As also shown in Fig. 2, tube 2 (which is made from a composition of twenty percent (20%) by weight BiO₃ and the remainder PEEK) is less radiopaque than tube 30. Those skilled in the art will appreciate that the desired degree of radiopacity (or conversely, radiolucence) may be obtained by varying the relative amounts of BiO₃ and PEEK in the composition from which a desired article is to be made.

Those skilled in the art will appreciate that the foregoing description of the invention is of a preferred embodiment and of certain specific alternative embodiments, and that various changes, modifications and other variations and adaptations thereof may be made without departing from the scope and spirit of the invention as set forth in the claims. The specific embodiments, and the dimensions, materials, and the like are merely illustrative and are not intended to limit the scope of the invention, as set forth in the claims.

## Claims

1. An implant for a mammal comprising an object comprising polyetheretherketone (PEEK) and at least ten percent (10%) by weight bismuth trioixide (BiO₃).

2. The implant according to claim 1 wherein said implant comprises a spinal cage.

3. The implant according to claim 1 wherein said implant comprises a dental device.

4. The implant according to claim 3 wherein the dental device comprises a filling of a tooth.

5. The implant according to any preceding claim wherein said implant is a long term implant.

6. The implant according to any preceding claim wherein said implant comprises a radiopaque marker.

7. An implant for a mammal comprising an item comprising BiO₃ and one or more polymers selected from the group consisting of polyetheretherketone, polyetherketone, polyetherketoneketone, polyphenylsulfone, and polyetherimide, wherein said implant comprises BiO₃ in an amount of between twenty percent (20%) to thirty percent (30%) by weight.

8. The implant according to claim 7 wherein said polymer comprises PEEK.

9. A medical device comprising at least ten percent (10%) by weight bismuth trioxide (BiO₃ and one or more polymers selected from the group consisting of polyetheretherketone, polyetherketone, polyetherketoneketone, polyphenlysulfone, and polyetherimide.

10. The medical device according to claim 9 wherein the device comprises a stent.

11. The medical device according to claim 9, wherein the device comprises a catheter.

12. The medical device or implant according to claim 1 or 9 wherein the medical device comprises a screw.

13. The medical device according to claim 9 wherein the medical device comprises a suture.

14. The implant or medical device according to any preceding claim wherein the mammal comprises a human being.

15. The implant or medical device according to any preceding claim wherein the implant further comprises at least twenty percent (20%) by weight BiO₃.

16. The implant according to claim 1 or 7 wherein the implant comprises thirty percent (30%) by weight BiO₃.

17. The medical device according to claim 10 wherein the device comprises from twenty percent (20%) to thirty percent (30%) by weight BiO₃.

18. The medical device according to claim 11 or 12 wherein the catheter or screw comprises at least about twenty percent (20%) by weight BiO₃.

19. The medical device according to claim 11 or 12 wherein the catheter or screw comprises from twenty percent (20%) to thirty percent (30%) by weight BiO₃.

20. A medical device comprising polytetrafluoroethylene (PTFE) and at least ten percent (10%) by weight bismuth trioxide (BiO₃).

21. The medical device according to claim 20 wherein said device comprises a portion made by paste extrusion.

22. The medical device according to claim 20 wherein said device comprises implant for a mammal.
